# NEW EUROPEAN PATENT SPECIFICATION

(11) **EP 0 816 511 B2**
(45) Date of publication and mention of the opposition decision: **14.06.2006**
(45) Mention of the grant of the patent: 22.01.2003
(21) Application number: 96113625.6
(22) Date of filing: 26.08.1996
(51) Int. Cl.: C12Q 1/02

(54) **Method of screening substances**
Verfahren zum Durchsuchen von Substanzen
Procédé de screening des substances

(30) Priority: 27.06.1996 EP 96110367
(43) Date of publication of application: 07.01.1998
(73) Proprietor: Clondiag Chip Technologies GmbH, 07743 Jena (DE)
(72) Inventor: Hinnen, Albert,Professor,Dr, 99510 Niederossla (DE)
(74) Representative: Maiwald Patentanwalts GmbH

(56) References cited:
- EP-A- 0 324 258
- EP-A- 0 608 722
- WO-A-91/02085
- WO-A-95/11969
- CURRENT OPINION IN BIOTECHNOLOGY, vol. 4, no. 4, 1 January 1993, pages 543-552, XP000196330 KIRSCH D R: "DEVELOPMENT OF IMPROVED CELL-BASED ASSAYS AND SCREENS IN SACCHAROMYCES THROUGH THE COMBINATION OF MOLECULAR AND CLASSICAL GENETICS"
- CURRENT GENETICS, vol. 25, no. 2, 1 February 1994, pages 95-100, XP000196332 VAHLENSIECK H F ET AL: "IDENTIFICATION OF THE YEAST ACC1 GENE PRODUCT (ACETYL-COA CARBOXYLASE) AS THE TARGET OF THE POLYKETIDE FUNGICIDE SORAPHEN A"
- Rine et al (1983) Proc. Acad. Natl. Sci. USA 80 p. 6750-6754.
- Baudin et al (1993) Nucleic acid research 21(14) p. 3329-3330.

## Description

### Background of invention

The availability of a rapidly increasing amount of genomic information is opening up new opportunities for expioitations. These are most prominent in areas which are strongly driven by biological knowledge, e.g. the medical sciences. Genomic research will lead to a better understanding of the molecular basis of many diseases and thus provides the basis for more specific and more effective drugs. In particular drug discovery programmes will profit from the availability of the rapidly growing arsenal of structurally identified genes. These genes and their products are important candidates for new drug targets. Genome structure analysis (DNA-sequencing) is progressing at a rapid pace and the first total genome analysis have been reported already. Among these the completion of the DNA-analysls of baker yeast's (*Saccharomyces cerevisiae*) is of particular interest since yeast represents the first eukaryotic organism whose genome is fully sequenced (press communication of German Federal Research Ministry, April 24, 1996). The conservation of basic structures and functions among eukaryotic organisms has already made yeast an important eukaryotic model organism from which extrapolations to higher eukaryotic cells (e. g. mammalian cells) are possible. Genomic research is likely to strengthen the role of yeast as a eukaryotic model organism.

A limiting factor in the exploitation of genomic information is the assignment of functions to known gene structures. Without this knowledge much of the available DNA-sequence information is only of limited value, i.e, it is only possible to make general conclusions as to their overall classification which are based on rough structural and/or functional criteria. Commonly used structural criteria are DNA-homologies and similarities of DNA and protein motifs to genes and gene products which are already known. Function assignments to new genes are more difficult. They usually depend on phenotypic changes based on genetic mutations. A general strategy for the latter is the study of gene "knockouts" (deletions or interruptions of protein coding regions, often called "open reading frames", ORF's). Gene disruption techniques are available for a number of organisms although the speed of obtaining specific disruptions varies a lotfrom one organism to another. In this respect the techniques developed for *S. cerevisiae* are highly advanced (Baudin et al., Nucleic Acids Res. 21 (1992), 3329).

Even for organisms like *S. cerevisiae* the assignment of functions to new and otherwise unidentifiable genes is a major task and will take several years for completion. In the meantime DNA sequence data will accumulate without the possibility to make appropriate use of it. There is therefore a need to develop systems which can make use of newly identified ORF's without an a priori knowledge of their cognate functions. If it were possible to find an approach suitable for exploiting ORF's irrespective of a detailed knowledge of their function this could enormously speed up their utility. In addition, if it were possible to detect the effect of chemical substances on their functions, such an approach could immediately be used to find new drugs interfering with theirfunction, to identify novel drug targets, and to monitor the biological responses of chemicals on a multitude of cellular targets.

Yeast has important advantages over other eukaryotic model organisms. It is easy to work with experimentally (fast growth, easy handling) and the knowledge about its biology is highly advanced (excellent genetic system, extensive biochemical and physiological know how, advanced cell biology). It has long been used as a biotechnological work horse. Since thousands of years wine and bread are classical products of the metabolic properties of whole yeast cells whereas ethanol and other defined metabolites are traditional biotechnology products ever since yeast is consciously used as production system. Since the beginning of the Eighties yeast is extensively used as host for recombinant gone products. Several recombinant proteins have been developed for pharmaceutical uses (e.g. vaccines, insulin, hirudin, etc.) which have already reached the market or are in advanced phases of clinical development.

In the advent of genomic research, yet another field of application is opening up for *S*. *cerevisiae:* yeast cells as models for.medicai uses. It has long been generally accepted that yeast is an ideal model organism for eukaryotic cell biology. Yeast has all the basic cellular structures and biochemical pathways of higher eukaryotes and shares the principal communication and signalling systems with mammalian cells. In many ways yeast cell biology fulfilled a pioneering role for understanding important eukaryotic cell functions such as biogenesis of organelles, cellular transport systems, signalling pathways, cell cycle, control of transcription, etc. Despite of this intimate structural and functional relatedness between the microbial yeast cell and the higher eukaryotic mammalian cell the utility of the yeast model for medical research has been limited, mainly because most human diseases were ill understood at the molecu lar and/or cellular level. At the latest since the inception of genomic research this is changing dramatically. Many monogenic diseases have been unravelled at the molecular level and many diseases with cellular origins are under intensive investigations. Again, yeast cells serve as a reference point. Since 1996, the entire genomic nucleotide sequence of *S*. *cerevisiae,* the first complete genome structure of a eukaryotic organism, is known. This marks a milestone in eukaryotic cell biology and is at the same time the starting point for a highly competitive international race for a new era of biological studies: the systematic assignment of functions to structurally defined genes. Due to the conserved nature of structures and furictions among eukaryotic organisms this will have immediate consequences for our understanding of eukaryotic cell biology as a whole (Tugendreich et al., Hum. Mol. Genet. 3 (1994), 1509). Available data confirm the notion that similarity In cellular structures are also reflected at the molecular level of DNA and proteins thus providing the possibility to engineer complex pathways and interaction systems In a modular fashion and exchanging them between different cellular systems. Again, yeast as an easily accessible organism will serve as a model for further investigations.

Taken together, yeast cells provide ideal systems to study pathological functions and pathways and/or to model such pathways (Kirsch, Curr. Opin. Biotechnol. 4 (1993), 543). The utility of the models will lie primarily in areas of medical diseases which have their origin in the malfunctioning of cellular processes (cancer, viral diseases, immunmodulation, etc.). Developing drugs on the basis of these molecular targets will lead to a different quality of medication replacing symptomatic treatment with causative cure.

In addition, the close relationship of *S. cerevisiae* with pathogenic fungi (e.g. *Candida albicans)* makes model building with the view to find novel antifungal drugs an extremely worthwhile exercise. This is particularly evident in the light of the marked world-wide increase of the incidence of fungal infections and the concomitant appearance of resistance to conventional therapeutic drugs.

Of course finding drugs which interfere with molecular pathways present or designed in yeast will not necessarily guarantee success at the level of a human patient Other criteria will have to be fulfilled to convert a biologically active compound into a medical drug. Among these are toxicological profile, pharmacological behaviour, drug targeting, etc.). Many of these parameters will have to be evaluated in secondary test systems such as higher eukaryotic cell lines and animal models. However, due to the simplicity of the screening approach the yeast models will make excellent primary screens.

Current screening approaches rely on various strategies (Bevan et ai., TIPTECH 13 (1995), 115). In the classical indication area antiinfectives the most straightforward approach is to rely on growth inhibition of the pathogenic organism itself. With this principle most antibiotics which are currently used in therapy have been identified. In recent years in most indication areas more sophisticated strategies have been followed which make use of defined molecular targets. Among these targets enzymes and other proteins (e.g. receptors) are most often used. The methods of genetic engineering have greatly facilitated target definition and development. Certain targets can readily be produced by genetic manipulations of microorganisms or higher eukaryotic cell lines to provide the basis for screening assays. Altematively, whole cell test systems can be improved by overexpression of specific target proteins (e. g. receptors). All of these approaches need an a priori definition of the envisaged target. This is only possible after an intensive phase of research into the biology of the target. There is therefore a distinct and long-felt need to develop novel screening systems with novel targets without the need to wait for more detailed information of the exact biological role of the targets. This need is particularly accentuated in the tight of rapid growth of DNA sequence information. Most of this information is not readily applicable although it is a rich source upon which biological knowledge can be built in the coming years. To use the available DNA sequence information now and in a more rapid and direct way is of crucial importance for modem drug discovery approaches.

### Detailed description of invention

The present invention offers a way capable of solving the need to detect the effect of substances on targets without a detailed knowledge of the individual targets. In another simple experimental procedure it offers a method suitable of identifying targets affected by drugs. More specifically, the invention consists of a method of screening substances which affect one or a multitude of targets or of identifying drug targets wherein drug targets designate molecular targets affected by drugs, comprising the steps of
- incubating in an ordered array cell cultures of S.cervisiae each overexpressing or, alternatively, underexpressing a specific target with pure substances or mixtures of substances,
- and identifying those substances exhibiting reduced growth inhibitory effects on specific overexpressing cell cultures as compared to reference (non-overexpressing) cell cultures or, alternatively, identifying those substances exhibiting reduced growth inhibitory effects on reference cell cultures as compared to specific underexpressing cell cultures and/or identifying the targets for specific substances wherein overexpression means changing gene expression levels by increasing gene copy numbers, or by increasing promoter strength as compared to the reference cell culture and wherein underexpression means using a single copy plasmid vector and decreasing promoter strength as compared to the reference cell culture or wherein underexpression means decreasing promoter strength as compared to the reference cell culture and wherein all targets essential for growth in S. cerevisiae are overexpressed or, alternatively, underexpressed in one array.

Accordingly, the method according to the invention is suitable to test for the biological effects of pure substances on specific targets, and likewise to identify molecular targets of specific substances having a known biological activity, especially drugs.

In its preferred embodiment, the invention presents a general solution to the problem of finding biologically active compounds which inhibit essential cellular functions of the cell used. The basic principle behind the invention is the specific inhibition of one or a few defined molecular targets thereby affecting growth. This is practically achieved by placing in an ordered array pure cell cultures each if them being transformed with one or a few specific genes which are overexpressed (underexpressed). Overexpression is obtained by introducing these genes on a multi-copy plasmid vector. Since cells which produce large quantities of specific gene products are less sensitive to specific drugs this will result in a differential growth pattem. Underexpression is obtained by bringing a gene under the control of a weaker (less effective) promoter on a single-copy plasmid vector. Thus, the method according to the invention allows for the development of a multitarget detection system which Is based entirely on the knowledge of available DNA-sequence data of the cell used. Because each possible target is overexpressed (underexpressed) individually and in a physically distinguishable manner, the biological effect of an isolated substance or substance mixture on a specific target can be detected and assigned directly and unambiguously.

The gist of the invention resides in the reversal of the known principle of genetic transformation as it has first been developed for microorganisms and later been applied for other organisms. Most transformation systems make use of a growth inhibitory substance and include as a "detoxifying agent" the gene coding for the target (selection marker) in the transforming DNA. Upon overexpression of the target as a result of DNA uptake, the inhibitor is effectively diluted and the transformed cell is made resistant and can survive. Instead of applying a specific drug together with an overexpressed target (selection marker) to obtain a resistant cell, the target is overexpressed in a specific cell line to identify an unknown drug. In other words, the known application of the phenomenon is to apply a known drug to a cell culture and to use the differential growth response of the cells towards this specific drug upon exposure to an increased gene dosage as a selection principle for transformation. Instead of this known use the novel use is based on one or a multitude of cell lines, with each individual cell line overexpressing one specific target. This arrangement will allow for the search of bioactive compounds which differentially inhibit specific cell lines as a result of their specific target overexpression status. In a variation of this procedure, known or unknown substances can be evaluated with respect to their molecular target. An extension of this approach lies in the possibility to create more sensitive cells by underexpressing of the target, e.g. as a result of modification of its transcriptional control sequences.

Cell lines suitable for performing the method according to the invention are cell lines of S. cesevisiae.

Since the entire yeast genome is sequenced, it is experimentally possible to construct a collection of yeast cells which is composed of individual cell lines each overexpressing (underexpressing) one specific known target (e.g. T1). All cell lines which express the other targets (T2,T3,T4, etc.) serve as reference point for target T1. If target T1 is hit and if T1 represents a gene product whose presence is essential for growth, the cell line overexpressing (underexpressing) target T1 will show a reduced (increased) growth resistance com- ' pared to the other cell lines. As a consequence, the action of a bioactive compound with an Inhibitory activity towards a specific target is indicated.

The technical realisation of the above invention is achieved if the individual cell lines overexpressing (underexpressing) specific targets are deposited in a ordered array. This makes automation possible and provides the basis for a robust high-throughput screening of chemical libraries and/or crude biological extracts. Such an array is a further embodiment of the present invention.

Accordingly, the invention concerns a solid support containing in an ordered array a multitude of cell lines each overexpressing or, alternatively, underexpressing a specific target optionally together with one or more reference cell lines.

A target can be any moiecuie which is enriched in a specific cell as a result of a transformation event. A target in the context of the present invention is, for example, a molecularly defined DNA segment, a gene, or a gene product which can interact with effector molecules and thus produce biological effects. Usually the target is the direct gene product (protein, RNA), although the target can in principle also be a close functional and/or structural interaction partner. In the most common situation the target is a protein. Especially, the target is a molecular target essential for growth of the cell.

In order to achieve overexpression of individual targets, each individual gene is cloned into a medium copy number vector (for *S. cerevisiae* this is preferably a 2µ vector) and transformed into a suitable host in a manner known in the art Underexpression is achieved by bringing an individual gene under the control of a weaker promoter and cloning it into a single-copy plasmid vector, or weakening the respective promoter by mutagenesis (deleting or changing part of the promoter sequence). Technically this is performed, for example, by replacing the promoter naturally linked to the gene in question to another, weaker promoter or promoter fragment applying conventional gene replacement or disruption techniques. In a variation of this approach specific genes may also be cloned under the control of a regulatable promoter thus providing specific conditions of gene overexpression or undsrexpression, respectively.

Cultures of individual transformants are placed in an ordered and predetermined way on a solid support. Preferred solid supports are commercially available microtiter dishes containing a defined number of wells, e. g. 96 wells. The cultures are preferably placed into the wells of such microtiter dishes, to produce a complete collection of strains. The solid support is ideally constructed in such a way to allow the preparation of replicas, e.g. by printing or pipetting. The solid supports will also contain control strains which are untransformed or transformed with the same vector without an additional gene insert.

The array of strains containing the clones overexpressing (underexpressing) the individual genes together with at least one reference strain is treated with solutions, especially aqueous solutions, of pure substances or substance mixtures, where substances are high molecular or, preferably, low molecular weight compounds from natural sources or synthetic or semisynthetic products.

Drugs are also comprised and may serve to identify the molecular targets affected thereby. After a growth period which allows differentiating growth inhibitory effects of the compounds growth yields are compared. If necessary, appropriate dilutions and incubation times have to be chosen to reach the sensitivity range. Those individual clones which are less (more) sensitive to compounds as compared to reference strains are identified as the hosts expressing the affected target.

For specific molecular targets with known inhibitors Internal standards can be included in the screening assays. The screening system may then be used for the search of additional inhibitors. However, only very few inhibitors of defined targets are known and knowledge of such inhibitors is not a prerequisite for the feasibility of the invention. Such compounds may merely be added to provide further evidence for its practicality and to include additional measures of internal control into the system. A preferred way of exploiting the invention is its use without standards and with molecular targets for which no known inhibitors exist. The most preferred application is its use as a multiple target screening and/or target identification system. In this arrangement a multitude of compounds or compound mixtures can be assessed simultaneously as to their effects on a known array of molecular targets. In a related procedure, this enables the identification of so far unknown molecular targets. This is of interest for the identification and development of novel targets for drug discovery and for the evaluation of the mode of action of known and unknown substances, e.g. toxic compounds, environmental pollutants, etc..

For the use in a multicomponent screening approach groups of genes belonging to biological classes may be formed. For example genes which code for structural elements of the cell wall or which are involved in the control of cell wall synthesis may be overexpressed (Kiis, Yeast 10(1994), 851). These genes are of particular interest for the identification of antifungal drugs. Other classes of genes may involve structural components of the cell architecture, genes controlling morphogenesis of organelies, protein transport and protein secretion, cell cycle, signal transduction, etc.. According to an estimation based on a large number of gene disruptions the yeast genome contains approximately 1000 gene which are essential for growth even under conditions of rich nutrient supply ("essential genes"). Many of these genes are not assigned to any biological functions, however compounds which inhibit the products of these genes are of great interest. Having all essential genes overexpressed (or underexpressed) in one array Is therefore of great vaiue.

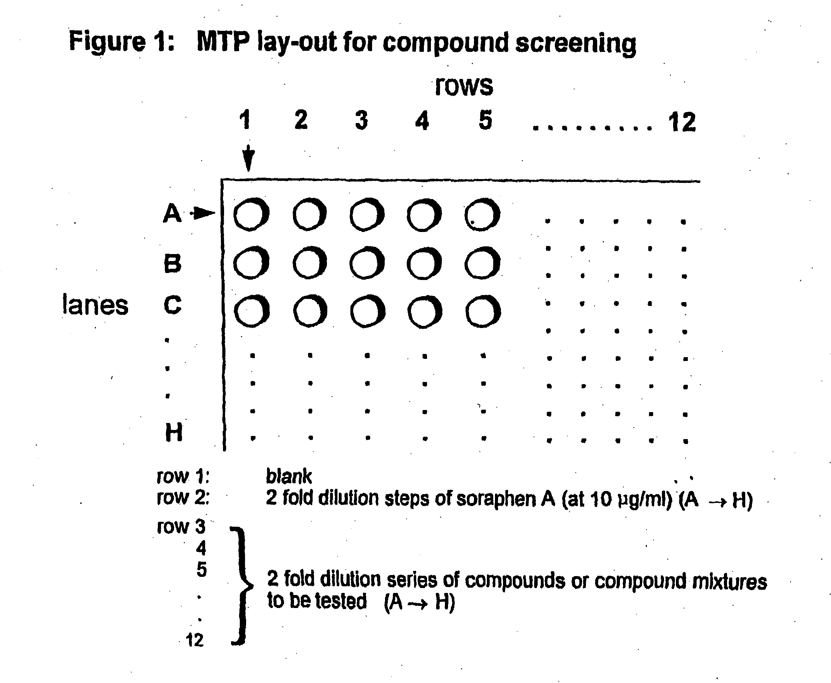

In the accompanying figure 1 a screening lay out on the basis of microtiter plates is shown.

The following examples serve to illustrate the invention but should not be construed as a limitation thereof.

### Examples

### 1. Development of a screening system for inhibitors of the acetyl-CoA carboxylase of S. cerevisiae

### 1.1 Preparation of a S. cerevisiae strain overexpressing acetyl-CoA carboxylase

The yeast ACC1 (FAS3) gene is contained on a 7.9 kb Sac I restriction fragment (Al-Feel et al., Proc. Acad. Sci. USA 89 (1992), 4534; Vahlensieck, Doctoral Thesis, University of Basle, 1993). This fragment is isolated from wild type yeast S288C using essentially the strategy described by Vahlensieck. The experimental protocols for the manipulation of DNA and plasmids and the growth of *Escherichia coli* cells are described (Sambrook etal., Molecular Cloning, Cold Spring Harbor Laboratory Press, 1989). Experimental protocols dealing with the manipulation of yeast DNA and plasmids as well as growth media and culture conditions are given in Sherman et al: (Methods in Yeast Genetics, Cold Spring Harbor Laboratory Press, 1983). Total chromosomal DNA from S288C is isolated, cut by restriction endonuclease Sad and applied to a 0.8 % agarose gel. DNA of the size of approximately 8 kb is isolated from the gel and ligated into the Sac I cut yeast 2µ vector pRS425 (Christianson et al., Gene 119 (1992),119). *E*. *coli* strain HB101 is transformed with aliquots from the ligation mixture using the ampR gene as a selection marker. A collection of *E*. *coli* colonies is obtained consisting of individual clones with specific vector-insert combinations. Forfurther processing, *E. coli* colonies are washed from the transformation plates and plasmid DNA is isolated. This yields a mixture of pRS425 plasmids with different inserts of yeast DNA. In orderto isolatefrom this mixture the specific hybrid plasmid containing the ACC1 gene the mixture is transformed into yeast strain GRF18 using the LEU2 gene of the vector as selection marker. The transformed yeast cells are screened for their resistance against the polyketide fungicide soraphen A (Vahlensieck et al., Curr. Genet. 25 (1994), 95) by growing individual transformants in liquid culture broth using YPD medium with 1 µg/ml soraphen A. Ovemight growth with incubation at 30°C reveals cultures with clear difference in cell densities: most yeast cultures show no growth, but a few cultures exhibit good growth and thus give indication for an increased gene dosage of the ACC1 gene. In order to verify this assumption total DNA from soraphen A resistant yeast cells is Isolated and *E. coli* strain HB101 is transformed as described above. Total plasmid DNA from individual *E. coli* transformants is isolated and analysed by restriction endonuclease digestion with Sac I to show the 7.9 kb insert and with EcoRI and KpnI to show diagnostic DNA fragment sizes. The product of this procedure is a hybrid pRS425 plasmsid having a 7.9 kb DNA insert containing the ACC1 gene. Transformation of this plasmid into yeast strain GRF18 yields yeast GRF18/ACC1 which is overexpressing the ACC1 gene as judged by its resistance towards the antibiotic soraphen A.

As a control GRF18 is transformed with plasmid pRS425 without insert (empty vector). This strain is called GRF18/TS.

### 1.2 Preparation of a screening lay out on the basis of microtiter plates (MTP)

Yeast strains GRF18/ACC1 and GRF18/TS are grown in YNB minimal medium supplemented with 20 µg/ml of L-histidine (YNB+his) to a density of 4x10⁷ cells/ml. The cell suspensions are 20-fold diluted with fresh YNB+his medium. 96-well MTP's (supplied by Greiner, Germany) are prepared which contain 10 µl of serially diluted compounds or compound mixtures per well as shown in figure 1. As a control one row only receives solvent (blank) and in one row a soraphen A (at 10 µg/ml) is serially diluted (standard). The incubation test is started by adding 100 µl aliquots of the 20-fold diluted cell suspension into the MTP wells. The MTP is incubated at 30°C for 1 - 2 days. At given intervals (6 to 10 hours) the MTP's are inspected by eye. Growth of the cells is compared between GRF18/T and GRF18/ACC1, blanks and soraphen A standards.

Altematively, cell growth can be assessed spectrophotometrically. If a compound or a compound mixture shows growth inhibitory activity for GRF18/TS but not or less for GRF18/ACC1 it is likely to include an inhibitor for acetyl-CoA carboxylase. The blanks and standards provide intemal controls for cell growth and acetyl-CoA carboxylase inhibition.

### 2. Development of a screening system for inhibitors of the arginine permease of S. cerevisiae

The cloning and overexpression of the yeast arginine permease is described by Broach et al. (Gene 8 (1979), 121). As described therein the yeast 2µ vector YEp13 is used for this purpose. In analogy to example 1, a MTP-assay is developed using the following pairs of yeast strains: GRF18/TC (=GRF18 transformed with YEp13) and GRF18/CAN1 (=GRF18 transformed with plasmid TLC-1 containing the gene coding for yeast arginine permease, see Broach et 61.). The test array is identical to the one described in example 1, except that canavanine, a known inhibitor of the arginine permease is used as a standard (Whelan et al., Genetics 91 (1979), 35).

### 3. Development of a multiple target screening and target identification system

Using the methods described in examples 1 and 2, any yeast gene for which an essential function for growth is known is subcloned, into yeast vector pRS425. Essential gene functions are determined using gene disruption techniques as described by Baudin et al. (see above). Using PCR techniques described therein, specific open reading frames (ORF's) are disrupted and the resulting phenotype is determined under in vivo conditions using classical genetic techniques (tetrad analysis, see Sherman et al.). Genes coding for essential functions are subcloned into the 2µ vector pRS425 using cloning techniques as described by Sambrook et al. (see above). Hybrid pRS245 vectors containing inserts coding for essential genes are transformed into yeast strain GRF18. As a result a collection of transformed GRF18 strains is created. These strains are grown in YNB+his medium as described in example 1, further processed as described, and arranged in rows of MTP's as described for individual transformants in examples 1 and 2.

### 4. Development of a screening system for inhibitors of acetyl-CoA carboxylase on the basis of ACC1 - underexpression

The ACC1 gene as described by Al-Feel et al. (Proc. Natl. Acad. Sci. USA 89 (1992), 4534) is taken to construct a GRF18 mutant strain which expresses the ACC1 gene from the *S. cerevisiae* CYC1 promoter. This is achieved by manipulating the ACC1 gene (7.9 kb Sacl restriction fragment) in such a way to insert the Smal-BamHI fragment comprising the CYC1 promoter of plasmid pLG6760-Z (Guarente, Methods in Enzymology Vol. 101 (1983), p. 181) into the HindIII site of the ACC1 gene (12 bp downstream from the initiator ATG from the ACC1 gene) by appropriate cloning and subcloning techniques (Sambrook et al., see above). The exact fusion of the CYC1 promoter to the ACC1 protein coding sequence is achieved by a PCR-directed deletion (Mullis, Ferre and Gibbs; PCR, The Polymerase Chain Reaction (1994), Birkhduser Boston). The linearized Sad fragment containing the ACC1 gene placed under the control of the CYC1 promoter is transformed into *S*. *cerevisiae* strain GRF 18 by cotransformation with plasmid Yep13. A 50-fold molar excess of the linearized fragment over the Yep13 vector is used; Transformants are screened for their sensitivity towards soraphen A. In order to vary the CYC1 expression levels glucose or lactose growth conditions are used (Guarente et al., Cell 36 (1984), 503). To verify the gene replacement Southern analysis is performed using the CYC1 gene and/or the ACC1 gene as a probe.

Correctly replaced mutant strains are chosen and used in screening assays with the unmutated *S. cerevisiae* GRF 18 strain as a control. Substances exhibiting stronger growth inhibition towards the mutated strain compared to the unmutated GRF 18 strain are excellent candidates for acetyl-CoA carboxylase inhibitors.

## Claims

1. A method of screening substances which inhibit one or a multitude of targets or of identifying drug targets wherein drug targets designate molecular targets inhibited by drugs, comprising the steps of
- incubating in an ordered array cell cultures of S. cerevisiae each overexpressing or, alternatively, underexpressing a specific individual target with pure substances or mixtures of substances,
- and identifying those substances exhibiting reduced growth inhibitory effects on specific overexpressing cell cultures as compared to reference (non-overexpressing) cell cultures or, alternatively, identifying those substances exhibiting reduced growth inhibitory effects on reference cultures as compared to specific underexpressing cell cultures, and thus identifying the targets for specific substances,
wherein overexpression means changing gene expression levels by increasing gene copy numbers, or by increasing promoter strength as compared to the reference cell culture and wherein underexpression means using a single copy plasmid vector and decreasing promoter strength as compared to the reference cell culture or wherein underexpression means decreasing promoter strength as compared to the reference cell culture, and wherein all targets essential for growth in S. cerevisiae are overexpressed or alternative underexpressed in one array.

2. Method of screening substances which inhibit one or a multitude of targets according to claim 1, comprising
- incubating in an ordered array cell cultures of S. cerevisiae each overexpressing a specific individual target with pure substances or mixtures of substances
- and identifying those substances exhibiting reduced growth inhibitory effects on specific overexpressing cell cultures as compared to reference cell cultures.

3. Method of identifying drug targets according to claim 1, comprising
- incubating in an ordered array cell cultures of S.cerevisiae each overexpressing a specific individual target with a drug,
- and identifying the targets for said drug.

4. Method according to claim 1 wherein the substance is a compound from natural sources or a synthetic or seniisynthetio compound.

5. Method according to claim 1 wherein the substance is a drug.

6. A solid support containing in an.ordered array a multitude of cell lines of S. cerevisial each overexpressing (underexpressing) a specific individual target optionally together with one or more reference cell lines, wherein overexpression means changing gene expression levels by increasing gene copy numbers, or by increasing promoter strength as compared to the reference cell lines and wherein underexpresssion means using a single copy plasmid vector and decreasing promoter strength as compared to the reference cell lines or wherein underexpression means decreasing promoter strength as compared to the reference cell lines, and wherein all targets essential for growth in S. cerevisiae are overexpressed or, alternatively, underexpressed in one array.

7. A solid support according to claim 9 wherein the support is a microtiter dish containing a defined number of wells.

## Patentansprüche

1. Ein Verfahren zum Durchsuchen von Substanzen, die eine oder eine Vielzahl von Zielstrukturen hemmen, oder zur Identifizierung von Wirkstoff-Zielstrukturen, wobei die Wirkstoff-Zielstrukturen molekulare Zielstrukturen kennzeichnen, die durch Wirkstoffe gehemmt werden, das Verfahren umfassend die Schritte
- Inkubieren von *S*. *cerevisiae* Zellkulturen in einer geordneten Anordnung mit reinen Substanzen oder Substanzgemischen, wobei jede Zellkultur eine spezifische Zielstruktur überexprimiert oder alternativ unterexprimiert,
- und Identifizieren der Substanzen, die im Vergleich zu Referenz (nichtüberexprimierenden)-Zellkulturen verringerte wachstumshemmende Wirkungen auf spezifische überexprimierende Zellkulturen zeigen, oder alternativ Identifizieren von Substanzen, die im Vergleich zu spezifischen unterexprimierenden Zellkulturen verringerte wachstumshemmende Wirkungen auf Referenzkulturen zeigen, und **dadurch** Identifizieren der Zielstrukturen für spezifische Substanzen,
wobei Überexpression eine Veränderung der Genexpressionsstärke durch Erhöhen der Genkopienzahl oder durch Erhöhen der Promotorstärke im Vergleich zu der Referenzzellkultur bedeutet, und wobei Unterexpression das Verwenden eines single-copy Plasmid-Vektors und das Erniedrigen der Promotorstärke im Vergleich zu der Referenzzellkultur bedeutet oder wobei Unterexpression das Erniedrigen der Promotorstärke im Vergleich zu der Referenzzellkultur bedeutet, und wobei alle für das Wachstum essentiellen Zielstrukturen in *S. cerevisiae* in einer Anordnung überexprimiert oder alternativ unterexprimiert werden.

2. Verfahren zum Durchsuchen von Substanzen, die die eine oder eine Vielzahl von Zielstrukturen hemmen, gemäß Anspruch 1, umfassend
- Inkubieren von *S. cerevisiae* Zellkulturen in einer geordneten Anordnung mit reinen Substanzen oder Substanzgemischen, wobei jede Zellkultur eine spezifische Zielstruktur überexprimiert
- und Identifizieren von Substanzen, die im Vergleich zu Referenzzellkulturen verringerte wachstumshemmende Wirkungen auf spezifische überexprimierende Zellkulturen zeigen.

3. Verfahren zur Identifizierung von Wirkstoff-Zielstrukturen gemäß Anspruch 1, umfassend
- Inkubieren von *S*. *cerevisiae* Zellkulturen mit einem Wirkstoff in einer geordneten Anordnung, wobei jede Zellkultur eine spezifische Zielstruktur überexprimiert,
- und Identifizieren der Zielstrukturen für den Wirkstoff.

4. Verfahren gemäß Anspruch 1, worin die Substanz eine Verbindung aus natürlichen Quellen oder eine synthetische oder halbsynthetische Verbindung ist.

5. Verfahren gemäß Anspruch 1, worin die Substanz ein Wirkstoff ist.

6. Ein fester Träger, der in einer geordneten Anordnung eine Vielzahl von *S. cerevisiae* Zelllinien, die jeweils eine spezifische individuelle Zielstruktur überexprimieren (unterexprimieren), optional zusammen mit einer oder mehreren Referenzzelllinien, enthält, wobei Überexpression eine Veränderung der Genexpressionsstärke durch Erhöhen der Genkopienzahl oder durch Erhöhen der Promotorstärke im Vergleich zu den Referenzzelllinien bedeutet, und wobei Unterexpression das Verwenden eines single-copy Plasmid-Vektors und das Erniedrigen der Promotorstärke im Vergleich zu den Referenzzelllinien bedeutet oder wobei Unterexpression das Erniedrigen der Promotorstärke im Vergleich zu den Referenzzelllinien bedeutet, und wobei alle für das Wachstum essentiellen Zielstrukturen in *S. cerevisiae* in einer Anordnung überexprimiert oder alternativ unterexprimiert werden.

7. Ein fester Träger gemäß Anspruch 9, wobei der Träger eine Mikrotiterschale mit einer definierten Anzahl von Vertiefungen ist.

## Revendications

1. Procédé de screening des substances qui inhibent une ou plusieurs cibles, ou d'identification de médicaments-cibles, dans lequel les médicaments-cibles désignent des cibles moléculaires inhibées par des médicaments, et qui comprend les étapes suivantes :
- incubation de cultures de cellules *S*. *cerevisiae* dans un arrangement organisé dont chacune sur-exprime ou, alternativement, sous-exprime une cible spécifique avec des substances pures ou de mélanges de substances,
- et identification de ces substances qui possèdent des effets réduits pour inhiber la croissance des cultures de cellules sur-exprimantes par comparaison avec des cultures de cellules de référence (non sur-exprimantes) ou, alternativement, identification de ces substances qui possèdent des effets réduits pour inhiber la croissance des cultures de référence par comparaison avec des cultures spécifiques de cellules sous-exprimantes, et ainsi identification des cibles pour des substances spécifiques,
dans lequel sur-expression signifie changement des niveaux d'expression des gènes en augmentant le nombre des copies de gènes ou en augmentant la force des promoteurs par comparaison avec la culture de cellules de référence, et dans lequel sous-expression signifie utilisation d'un vecteur plasmide « single copy » et réduction de la force des promoteurs par comparaison avec la culture de cellules de référence ou dans lequel sous-expression signifie réduction de la force des promoteurs par comparaison avec la culture de cellules de référence, et dans lequel toutes les cibles dans *S*. *cerevisiae* essentielles pour la croissance sont sur-exprimées ou, alternativement, sous-exprimées dans un arrangement.

2. Procédé de screening des substances qui inhibent une ou plusieurs cibles selon la revendication 1, comprenant
- l'incubation de cultures de cellules *S. cerevisiae* dans un arrangement organisé dont chacune sur-exprime une cible spécifique avec des substances pures ou un mélange de substances,
- et l'identification de ces substances qui possèdent des effets réduits pour inhiber la croissance des cultures de cellules sur-exprimantes par comparaison avec des cultures de cellules de référence.

3. Procédé d'identifier des médicaments-cibles selon la revendication 1, comprenant
- l'incubation de cultures de cellules *S. cerevisiae* dans un arrangement organisé dont chacune sur-exprime une cible spécifique avec un médicament,
- et identification des cibles pour ledit médicament.

4. Procédé selon la revendication 1, dans lequel la substance est un composé de sources naturelles ou un composé synthétique ou semi-synthétique.

5. Procédé selon la revendication 1, dans lequel la substance est un médicament.

6. Un porteur solide contenant, dans un arrangement organisé, plusieurs lignes de cellules *S*. *cerevisiae* dont chacune sur-exprime (sous-exprime) une cible spécifique individuelle en option avec une ou plusieurs lignes de cellules de référence, dans lequel sur-expression signifie changement des niveaux d'expression des gènes en augmentant le nombre des copies de gènes ou en augmentant la force des promoteurs par comparaison avec la ligne des cellules de référence, et dans lequel sous-expression signifie utilisation d'un vecteur plasmide « single copy » et réduction de la force des promoteurs par comparaison avec les lignes de cellules de référence ou dans lequel sous-expression signifie réduction de la force des promoteurs par comparaison avec les lignes de cellules de référence, et dans lequel toutes les cibles dans *S. cerevisiae* essentielles pour la croissance sont sur-exprimées ou, alternativement, sous-exprimées dans un arrangement.

7. Un porteur solide selon la revendication 9, dans lequel le porteur est une plaque de microtitrage contenant un nombre défini de puits.
